# EUROPEAN PATENT APPLICATION

(11) **EP 3 960 297 A1**
(43) Date of publication of application: **02.03.2022**
(21) Application number: 21193483.1
(22) Date of filing: 27.08.2021
(51) Int. Cl.: B01L 9/00, G01N 33/00, G01N 35/00, B01L 3/00

(54) **MICROFLUIDIC DEVICE FOR IMAGE MULTIPLEXING**

(30) Priority: 28.08.2020 US 202017005470; 02.02.2021 US 202117165424
(71) Applicant: Leica Microsystems CMS GmbH, 35578 Wetzlar (DE)
(72) Inventor: CORWIN, Alex David, Niskayuna, New York, 12309-1027 (US); FOGELBERG, John Tenny, Issaquah, Washington, 98027 (US); SURRETTE, Christine Lynne, Niskayuna, New York, 12309-1027 (US); MCDONOUGH, Elizabeth, Niskayuna, New York, 12309-1027 (US); HAMMOND, Tyler, Niskayuna, New York, 12309-1027 (US); PETERSON, Sara, Niskayuna, New York, 12309-1027 (US); UNWIN, Joseph Allen, Issaquah, Washington, 98027 (US)
(74) Representative: DehnsGermany Partnerschaft von Patentanwälten

(57) **Abstract**

The present invention relates to a microfluidic device 100, 400, 500, 600 for image multiplexing. The microfluidic device 100, 400, 500, 600 comprises a base structure 110 comprising an optical window 140 and a fluid well insert 120 coupled to the base structure 110. The fluid well insert 120 is configured to retain a microscope slide 130 for mounting of a biological sample 150 within the microfluidic device 100, 400, 500, 600. The fluid well insert 120 is also configured to provide a fluid to said biological sample 150. A fluid well insert lid 160 coupled to the fluid well insert 120 is also provided.

## Description

### Cross-reference to Related Applications

The present application is a Continuation-in-Part of US Application Serial No. 17/005,470 filed on August 28, 2020, the entire contents of which are hereby incorporated by reference.

### Field of the Invention

The present invention relates generally to a microfluidic device for multiplex staining and imaging which enables a technique for encapsulating a mounted biological sample so as to allow for sequential *in situ* multiplexed image analysis of the sample based on the concept of dye cycling.

### Background

For sequential *in situ* multiplexed image analysis, a biological sample such as a tissue sample or tissue microarray (TMA) needs to be stained with multiple molecular probes to investigate protein expression or spatial distribution quantitatively or qualitatively, see, for example, US 7,629,125 and US 7,741,046, which are hereby incorporated by reference in their entirety herein to the maximum extent permitted. The staining process may be performed manually or by using an automated slide stainer. Conventionally, such methods may require the use of a coverslip to allow for imaging of the stained sample and to provide physical protection therefor.

However, given various drawbacks associated with the use of coverslips, such as the need to manually remove them when the sample needs to be exposed to various reagents in-between various process steps, certain systems have been developed which can eliminate the need to use coverslips when imaging such biological samples. For example, various such systems are disclosed in US 2009/0253163 A1 and US 2014/0055853 A1, which are also hereby incorporated herein by reference in their entirety to the maximum extent permitted.

Additionally, in various configurations, the systems of US 2009/0253163 A1 and US 2014/0055853 A1 may incorporate, for example, a Cell DIVE^{™} instrument which provides a standardized automated staining, imaging and image processing workflow for multiplex imaging of slides, and which is commercially available from Cytiva^{™}, Global Life Sciences Solutions USA LLC, 100 Results Way, Marlborough, MA 01752, United States of America.

Nevertheless, whilst such conventional systems provide a significant improvement upon preceding systems, there is a continuous desire to improve the usability, speed/throughput, accuracy and sensitivity of systems that are used to perform multiplexed image analysis of biological samples.

Hence, the present invention, as defined by the appended claims, is provided.

### Summary of Invention

According to a first aspect, the present invention provides a microfluidic device for image multiplexing. The microfluidic device comprises a base structure comprising an optical window and a fluid well insert coupled to the base structure. The fluid well insert is configured to retain a microscope slide for mounting of a biological sample within the microfluidic device. The fluid well insert is also configured to provide a fluid to said biological sample. A fluid well insert lid coupled to the fluid well insert is also provided.

According to a second aspect, the present invention provides a microfluidic device for image multiplexing comprising a closed top fluid well insert attached to a base structure. The base structure is configured to retain a microscope slide within the microfluidic device, in particular with the help of rotatable eccentric cams. The closed top fluid well insert is configured to form a fluid tight seal with the microscope slide when attached to the base structure.

The biological sample may be a sample obtained from a biological subject, including a sample of biological tissue or of fluid origin obtained *in vivo* or *in vitro.* Such samples may be, but are not limited to, tissues, fractions, and cells isolated from mammals including, humans.

By way of the fluid well insert, a fluid well may be provided adjacent to the biological sample. Advantageously, such a fluid well may be shaped and dimensioned such that it can substantially reduce fluid contact with any component that covers the well so as to reduce and/or prevent the formation of bubbles, foam, or the like within the fluid well in proximity to the biological sample. Such a fluid well insert thus enables improved imaging to be provided when using the microfluidic device and further also reduces the need to introduce the fluid therein under high pressures which might damage the biological sample.

Various other advantages of certain aspects and embodiments of the present invention are also envisaged, and will become apparent from the description that follows.

### Brief Description of the Drawings

In the drawings:
Figure 1 shows a microfluidic device in accordance with a first embodiment of the present invention showing a covered fluid well insert attached to a base structure;
Figure 2 shows the microfluidic device of Figure 1 with the covered fluid well insert removed from the base structure;
Figure 3 shows the microfluidic device of Figure 1 in a disassembled configuration;
Figure 4 shows the fluid well insert of Figure 3 attached to the base structure in plan view;
Figure 5 shows the microfluidic device of Figure 1 in a disassembled configuration;
Figure 6 shows the microfluidic device of Figure 1 in an exploded view;
Figure 7 shows an alternative fluid well insert for use in various embodiments of the present invention;
Figures 8A to 8C schematically show how a fluid well insert cavity may be aspirated using various embodiments of the present invention;
Figure 9 shows a graph illustrating how fluid depth in the fluid well insert cavity of an embodiment of the invention is related to the fluid volume therein.
Figure 10 shows a microfluidic device in accordance with a second embodiment of the present invention in an exploded view showing a closed top fluid well insert attachable to a base structure;
Figure 11 shows a cutaway view of the microfluidic device of Figure 10 showing the closed top fluid well insert attached to the base structure;
Figure 12 shows a plan view of the microfluidic device of Figure 10 showing the closed top fluid well insert attached to the base structure;
Figure 13 shows a microfluidic device in accordance with a third embodiment of the present invention in exploded view showing a fluid well insert configured to provide a fluid well cavity with an adjustable and/or switchable volume; and
Figure 14 shows a plan view of the microfluidic device of Figure 13 showing the fluid well insert attached to the base structure.
Figure 15 shows a cutaway view of another embodiment of a microfluidic device showing a non-contact dispenser system comprising a reciprocating syringe.

### Detailed Description

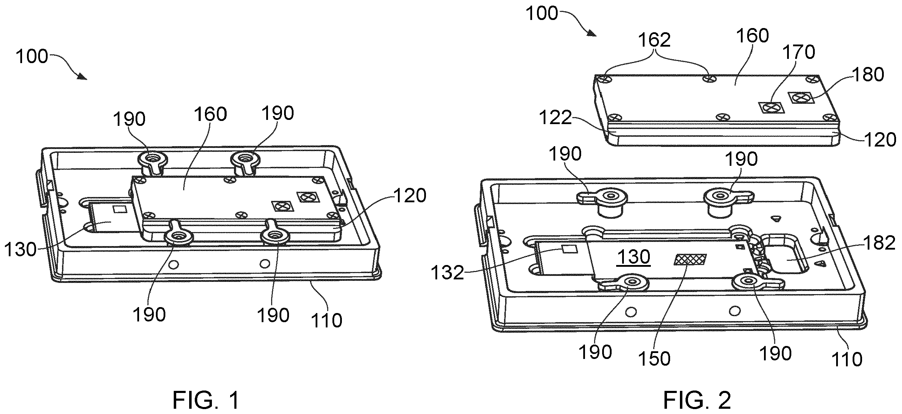

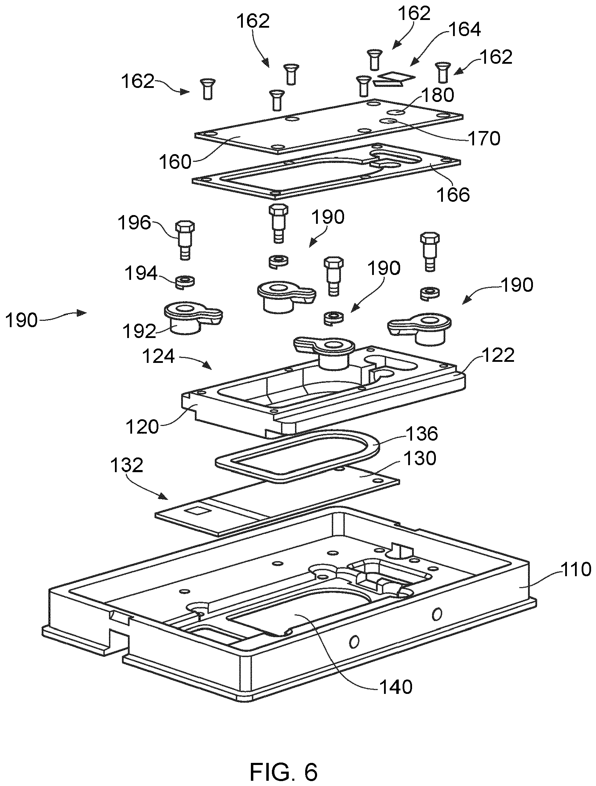

Figure 1 shows a microfluidic device 100 in accordance with a first embodiment of the present invention showing a covered fluid well insert 120 attached to a base structure 110. The base structure 110 includes an optical window 140 (see Figure 6, for example). Fluid well insert 120 is covered by fluid well insert lid 160, which may, for example, be affixed thereto by screws 162 or the like. Preferably, the fluid well insert lid 160 comprises an opaque cover material. Such an opaque fluid well insert lid 160 thereby provides protection from background light intrusion during imaging, which can otherwise wash out the image(s) taken via the optical window 140.

Various imaging techniques may be used. For example, the biological sample may sequentially be: i) stained with a dye, ii) imaged with a high resolution microscope or fluorescent reporter, and iii) bleached or quenched, with the cycle i)-iii) then being repeated as necessary.

The base structure 110 comprises a recess for retaining a microscope slide 130 therein. The fluid well insert 120 can be coupled to the base structure 110 by way of quick release coupling mechanisms 190, and is thereby configured to retain the microscope slide 130 in the recess and provide a fluid well insert cavity 124 adjacent thereto. In use, a biological sample 150 is provided on the microscope slide 130 on a surface thereof adjacent to the fluid well insert 120 and within the fluid well insert cavity 124. The biological sample 150 is then imaged via the optical window 140.

Preferably, a portion of the microscope slide 130 remains uncovered by, and is visible adjacent to, the fluid well insert 120 when the latter is *in situ,* such that a labelled portion 132 remains visible during the imaging process. The labelled portion 132 may then be used to uniquely identify the biological sample 150, for example, by way of a 2-D barcode, barcode, printed text, or the like, so as to aid in automated sample processing.

The microfluidic device 100 thus provides a closed well device for housing a portion of a microscope slide with a sealed cover. Such an arrangement advantageously prevents drying and contamination of the biological sample 150. Moreover, the cover and well geometry can also provide for improved efficiency fluid dispensing and aspiration without the need to remove any covers, as will be further discussed below.

Figure 2 shows the microfluidic device 100 of Figure 1 with the covered fluid well insert 120 removed from the base structure 110. The fluid well insert 120 is covered by the fluid well insert lid 160 to which it is attached by means of six screws 162. Fluid well insert lid 160 further comprises a fluid inlet port 170 and a fluid aspiration port 180. Such ports 170, 180 may comprise respective slotted membranes 164 formed from resilient material, that can accommodate a pipette or syringe therein, as are known in the art. Such pipettes or syringes may be used to introduce or extract fluid(s) into or from the microfluidic device 100, for example, as part of a robotically controlled imaging process (e.g. using a robotically coupled stainer and imager, such as a Kinetix^{®} based robot system available from Rockwell Automation, Milwaukee WI, USA). Multiplexing reagents may also be dispersed from a dispenser (not shown) provided in fluid communication with the fluid inlet port 170.

The microscope slide 130 is shown in the recess of the base structure 110. The biological sample 150 is provided on an area of the microscope slide 130 adjacent to the labelled portion 132. The recess of the base structure 110 is also formed adjacent to a fluid aspiration cavity 182 which is fluidically coupled to the fluid aspiration port 180 when the fluid well insert 120 is *in situ.*

Four quick release coupling mechanisms 190 are provided. In Figure 2, these are shown in a released position. However, when the fluid well insert 120 is *in situ* (as shown in Figure 1), the quick release coupling mechanisms 190 are configured to engage with a ledge portion 122 of the fluid well insert 120 to hold it in place. The quick release coupling mechanisms 190 may be provided as swivel locks that enable efficient replacement of the microscope slide without the need to use any tools.

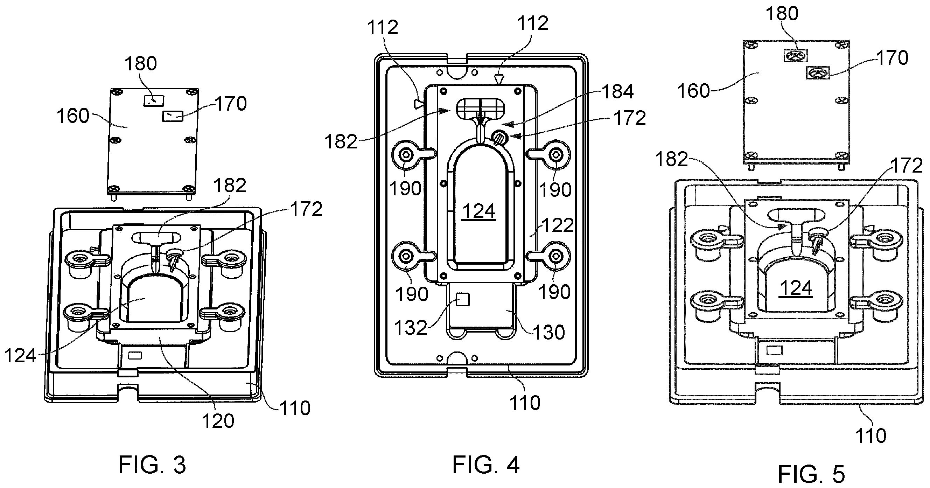

Figure 3 shows the microfluidic device 100 of Figure 1 in a disassembled configuration. Fluid well insert lid 160 is shown as being removed, whilst the fluid well insert 120 is shown *in situ,* being attached to the base structure 110 by way of the quick release coupling mechanisms 190.

Fluid well insert 120 provides a fluid well insert cavity 124 adjacent to the portion of the microscope slide 130 that is used to support a biological sample 150. The fluid aspiration cavity 182 is also shown fluidically connected to the fluid well insert cavity 124.

A fluid dispenser cavity 172 is also provided connected to the fluid well insert cavity 124. When the fluid well insert lid 160 is attached, the fluid inlet port 170 thereof is provided adjacent to the fluid dispenser cavity 172 in fluid communication therewith.

In various preferred embodiments, one or more channels connecting the fluid inlet port 170 with the fluid dispenser cavity 172 can be provided. Such a channel(s) may be oriented such that the fluid flow path from fluid dispenser cavity 172 towards the biological sample 150 is indirect (e.g. provides an off-axis fluid entry point) so as to minimise the disturbance to the biological sample 150 either from the force of any dispensed liquid or from any accidental contact with a pipette/syringe tip. By providing an off-axis fluid entry point/port it is possible to maintain uniformity of the, optionally opaque, fluid well insert lid 160 above the microscope slide 130. In contrast, were a fluid entry port to be provided above the microscope slide 130, it is necessary to account for possible optical differences and stray light induction due to the differences formed in the insert lid material (e.g. opaque material versus an opening).

Figure 4 shows the fluid well insert 120 of Figure 3 attached to the base structure 110 in plan view. The base structure 110 comprises fiducial features 112 thereon. The fiducial features 112 may aid in placement of the microscope slide 130 and in aligning images thereof during the imaging process(es).

Also depicted in Figure 4 is a fluid guide structure 184 provided between the fluid well insert cavity 124 and the fluid aspiration cavity 182. The fluid guide structure 184 is configured to help channel fluid into the fluid aspiration cavity 182 as the micro fluidic device 100 is tilted. For example, tilting (either manual or automated) may be used to help with the aspiration process during aspiration of the microfluidic device 100 during a multiple stage image acquisition process.

In various embodiments, the fluid guide structure 184 may comprise a shaped (e.g. sloping) floor portion and/or a fluid dam structure configured to enable the fluid aspiration cavity 182 to retain fluid therein should the microfluidic device 100 be untilted. The fluid aspiration cavity 182 may itself incorporate a sloped floor portion (e.g. provided in a plane that is non-parallel to that of the plan view of Figure 4), a stepped floor, or the like, to aid in preventing fluid flowing back into the fluid well insert cavity 124 during any untilting of the microfluidic device 100. For example, the fluid aspiration cavity 182 may be provided with a fluid dam that serves to keep fluid trapped, and the height of the fluid dam can be used to predefine a range of tilt angles for which fluid remains trapped therein.

Furthermore, various shaped features (e.g. one or more wing shaped features for enabling low profile tilting of the microfluidic device 100) may also be provided within the fluid well insert cavity 124 to direct fluid towards the fluid aspiration cavity 182 as the microfluidic device 100 is tilted/rocked.

Figure 5 shows the microfluidic device 100 of Figure 1 in a disassembled configuration as per Figure 3. One further advantage of various embodiments of the present invention lies in the fact that the fluid well insert cavity 124 can be made relatively spacious (e.g. with a relatively low cross-sectional aspect ratio, e.g. a width to height ratio and/or length to height ratio of less than 10:1, 5:1, 3:1, 2:1, etc.), such that the fluid well insert lid 160 can be distanced from the sample. This permits the microfluidic device 100 to be operated using relatively low pressure fluids, and avoids the need to use high pressure fluid inputs which can cause sample/tissue damage and which require the use of more expensive components, e.g. pumps, and that are more prone to increased chances of leakage etc.

In one embodiment, the fluid well insert cavity 124 has a width of approximately 22 mm, a length of approximately 48 mm and a maximum depth/height of about 9 mm. A sloped wall portion may also be formed in the fluid well insert cavity 124 adjacent to a floor portion thereof, sloping into the fluid well insert cavity 124 towards the centre thereof. For example, a substantially 45° sloped wall portion may be provided extending approximately 3 mm into the fluid well insert cavity 124. Fluid may thus be provided to the top of the sloped wall portion to a depth of about 3 mm, with the fluid well insert cavity 124 having an approximate width to height ratio of 22:9 (2.4:1) and an approximate length to height ratio of 48:9 (5.3:1) respectively.

The configuration of the present invention can be used to reduce the formation of bubbles between the biological sample 150 and the fluid well insert lid 160 which can otherwise cause non-uniform staining of the biological sample 150 and thereby degrade images by introducing random image artefacts. The fluid well insert lid 160 further provides protection for the biological sample by maintaining humidity and preventing contamination during fluid treatment, imaging, transportation and storage steps.

Figure 6 shows the microfluidic device 100 of Figure 1 in an exploded view. Base structure 110 includes an optical window 140 therein. In this embodiment, the optical window 140 is formed as an open aperture in the base structure 110. The optical window is thus substantially transparent to electromagnetic radiation in at least one of the infrared, near-infrared, visible and/or ultraviolet spectra. However, in alternative embodiments, the optical window 140 may comprise one or more window materials for transmitting radiation at any desired wavelength, or spectra of wavelengths. The base structure also includes a recess for retaining a microscope slide 130 therein.

A first gasket 136 is provided between the microscope slide 130 and the fluid well insert 120. First gasket 136 provides a fluid-tight seal between the microscope slide 130 and the fluid well insert 120 so as to prevent fluid from escaping from the fluid well insert cavity 124. Four quick release coupling mechanisms 190 are also provided at respective corners of the fluid well insert 120 for coupling the fluid well insert 120 to the base structure 110. Each respective quick release coupling mechanism 190 incorporates a rotatable lug 192 resiliently fastened to the base structure 110 using a spring 194 and fastener 196. The spring 194 and fastener 196 thus provide a respective spring clamp attached to the base structure 110.

When rotated to engage a ledge portion 122 of the fluid well insert 120, the rotatable lugs 192 are biased into engagement therewith by respective of the springs 194. Hence, spring-loaded swivel locks are provided that enable the rapid replacement of sample-bearing microscope slides whilst also ensuring a fluid-tight seal is provided between the fluid well insert cavity 124 and the microscope slide 130.

A second gasket 166 is provided between the fluid well insert lid 160 and the fluid well insert 120 to prove a fluid-tight seal therebetween. Screws 162 are used to affix the fluid well insert lid 160 to the fluid well insert 120. Respective slotted membranes 164 are provided in the fluid inlet and fluid aspiration ports 170, 180 to provide pierceable seals therein.

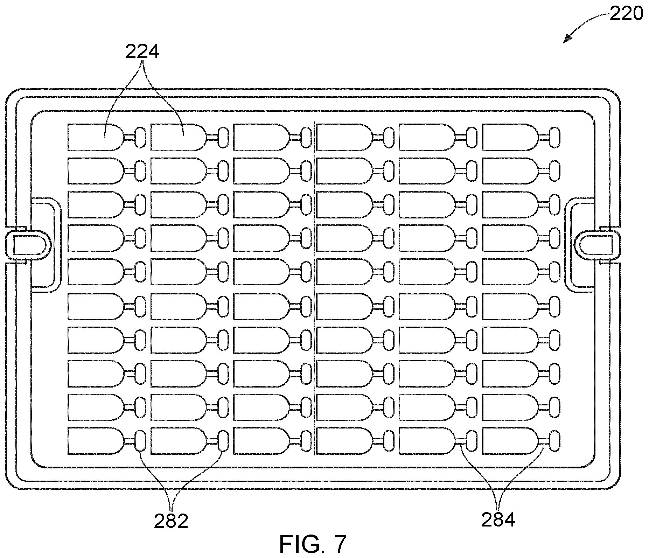

Figure 7 shows an alternative fluid well insert 220 for use in various embodiments of the present invention. The fluid well insert 220 comprises an array of respective fluid well insert cavities 224 provided in a grid-like arrangement. Each fluid well insert cavity 224 can be filled and aspirated using respective associated fluid input and fluid aspiration ports (not shown). Each fluid well insert cavity 224 is further fluidically coupled to a respective fluid aspiration cavity 282 via a fluid guide structure 284.

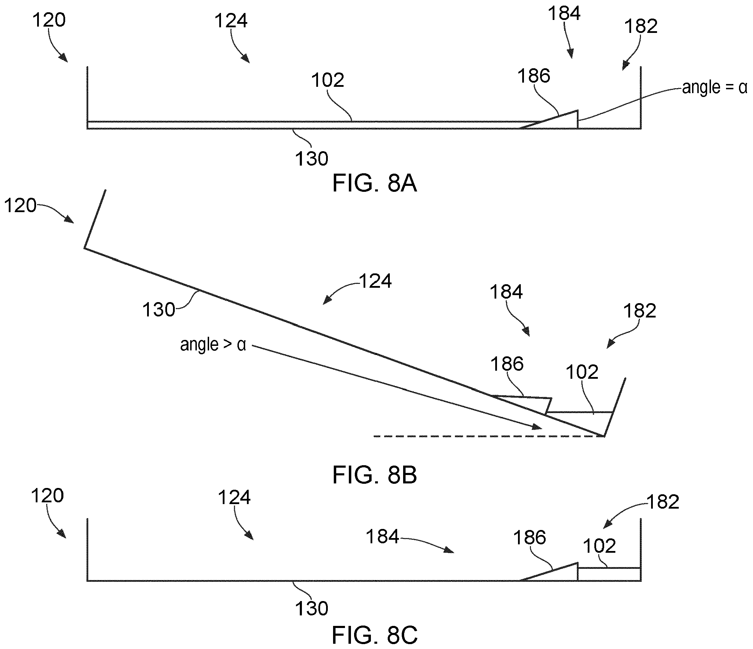

Figures 8A to 8C schematically show how the fluid well insert cavity 124 may be aspirated using various embodiments of the present invention.

Figure 8A shows the fluid well insert cavity 124 when the microfluidic device 100 is in an untilted state with a fluid 102 provided therein. The fluid 102 is in contact with the biological sample 150, and may have a volume of about 50 µl, for example. The fluid well insert cavity 124 is provided by the fluid well insert 120 and is sealed at the bottom thereof by coupling to the microscope slide 130.

The fluid 102 is prevented from entering the fluid aspiration cavity 182 by way of fluid guide structure 184. Fluid guide structure 184 comprises a fluid dam structure 186 therein. The fluid dam structure 186 is formed as a wedge and has a substantially triangular cross sectional shape. Preferably, the substantially triangular cross sectional shape provides a sloped surface portion thereof angled at an angle (α) from about 10° to about 15° with respect to a floor portion of the fluid well insert 120/microscope slide 130. The height of the dam structure 186 may be chosen such that a predetermined amount of fluid can be retained in the fluid aspiration cavity 182 after tilting.

Figure 8B shows the fluid well insert cavity 124 when the micro fluidic device 100 is in a tilted state. The microfluidic device 100 has been tilted in this instance to an angle that is greater than that of the sloped surface portion of the dam structure 186 (i.e. > α).

Such a tilting action causes the fluid 102 to run over the dam structure 186 and to accumulate in the fluid aspiration cavity 182.

Figure 8C shows the fluid well insert cavity 124 when the microfluidic device 100 has been returned to an untilted state after being tilted as per Figure 8B. In this instance, as the microfluidic device 100 is untilted, the dam structure 186 blocks the return of fluid 102 from the fluid aspiration cavity 182 to the fluid well insert cavity 124 such that it is retained therein. Subsequently, the fluid 102 may then be aspirated from the fluid aspiration cavity 182.

In various embodiments of the invention, a jig (or positioning stand) (not shown) may be provided which is configured to hold the microfluidic device 100 in an untilted or tilted position to improve handling of the microfluidic device 100, specially during aspirating and dispensing of fluid from and into the fluid well insert cavity 124. The jig (not shown) could be used for automatic, semiautomatic and/or manual operation of the microfluidic device 100.

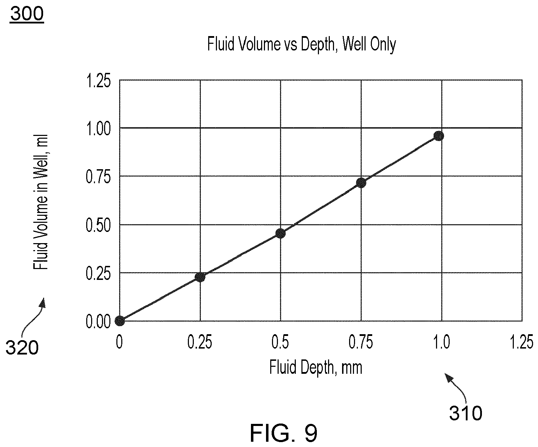

Figure 9 shows a graph 300 illustrating how fluid depth 310 in the fluid well insert cavity 124 of an embodiment of the invention is related to the fluid volume 320 therein. In various embodiments, the fluid well insert lid 160 is configured to be spaced at a distance that is at least twice the maximum depth of fluid 102 that is to be provided in the fluid well insert cavity 124 to help prevent the formation of bubbles therein.

As is apparent from Figure 9, the volume of fluid 320 in the fluid well insert cavity 124 varies linearly with the fluid depth 310 therein.

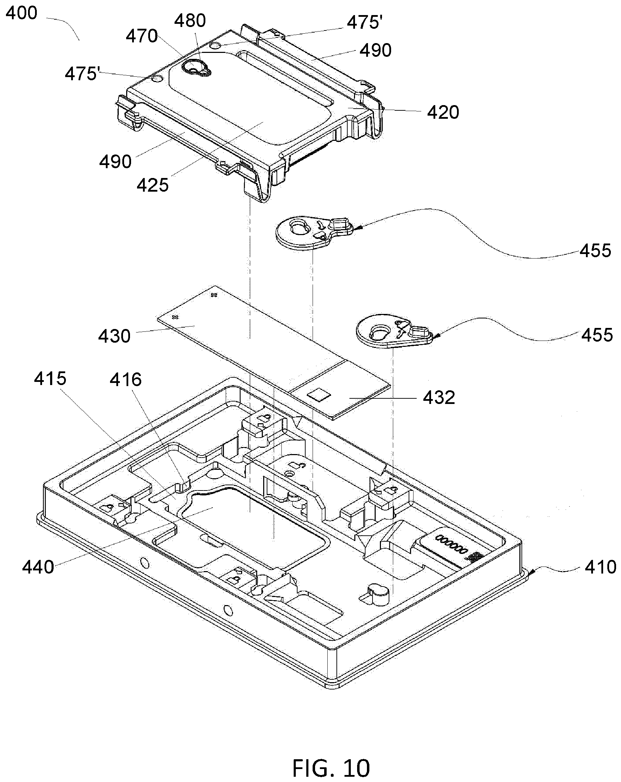

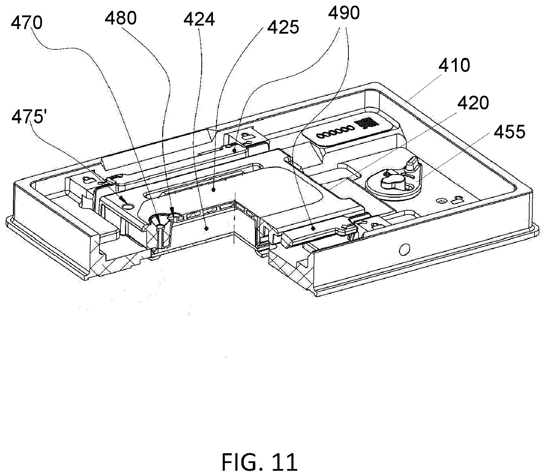

Figure 10 shows a microfluidic device 400 in an exploded view in accordance with a second embodiment of the present invention, illustrating a closed top fluid well insert 420 that is attachable to a base structure 410. The closed top fluid well insert 420 is a single component which is closed on top and thus does not require attachment of a separate lid component during use of the microfluidic device 400. The closed top fluid well insert 420 comprises an integral fluid well insert cavity 424 (shown in Figure 11) that is located under a top surface 425 of the closed top fluid well insert 420. The closed top fluid well insert 420 being a single component can be easily manufactured by any suitable low-cost manufacturing methods such as injection moulding, die casting or 3D printing.

The closed top fluid well insert 420 also comprises one or more integrated fittings 490 for releasably coupling the closed top fluid well insert 420 to the base structure 410. Figure 10 shows two integrated fittings 490 provided, respectively, along two opposite sides of the closed top fluid well insert 420. The two integrated fittings 490 may be provided as snap fittings in the form of flexible wing structures. In alternate embodiments of the invention, the one or more integrated fittings 490 could be of a different type, shape or design.

The closed top fluid well insert 420 further comprises an integrated seal (not shown) that provides a fluid-tight sealing between a microscope slide 430 and the fluid well insert cavity 424 when the closed top fluid well insert 420 is coupled to the base structure 410 using the one or more integrated fittings 490. The integrated seal prevents leakage of fluid contained within the fluid well insert cavity 424 of the closed top fluid well insert 420 when the microfluidic device 400 is in use. The integrated seal could be injection moulded as an over-mould during the manufacturing of the closed top fluid well insert 420 or provided by any other suitable method. In various embodiments, the seal could be provided in the form of a separate rubber gasket.

The closed top fluid well insert 420 also comprises one or more fluid aspirate/dispense ports 470 and one or more vents 480 located on the top surface 425 of the closed top fluid well insert 420. The one or more fluid aspirate/dispense ports 470 and the one or more vents 480 are in fluid communication with the fluid well insert cavity 424 located under the top surface 425 of the closed top fluid well insert 420 to allow fluid transport into and out of the fluid well insert cavity 424 during a multiplex staining and imaging operation.

When a fluid is dispensed into the fluid well insert cavity 424 through the one or more fluid aspirate/dispense ports 470, said fluid displaces the air within the fluid well insert cavity 424. As more fluid is being dispensed, more air gets displaced causing the pressure inside the fluid well insert cavity 424 to start building up. Pressure build-up inside the fluid well insert cavity 424 could eventually cause said fluid to be pushed back out of the one or more fluid aspirate/dispense ports 470, thereby causing a leakage from the microfluidic device 400. The one or more vents 480 allow the displaced air to escape from the fluid well insert cavity 424, thereby allowing to balance the pressure inside the fluid well insert cavity 424 with the pressure outside the fluid well insert cavity 424 when the microfluidic device 400 is in use. Thus, one advantage of the one or more vents 480 is that they make the microfluidic device 400 leak resistant.

Figure 10 shows one fluid aspirate/dispense port 470 located on the top surface 425 near an end portion of the closed top fluid well insert 420. Figure 10 also shows one vent 480 located adjacent to the fluid aspirate/dispense port 470 on the top surface 425 of the closed top fluid well insert 420. In this embodiment, the fluid aspirate/dispense port 470 is positioned at a sufficiently distant location from a biological sample enclosed within the fluid well insert cavity 424. This positioning of the fluid aspirate/dispense port 470 is thus advantageous in that it prevents disturbing or accidentally poking the biological sample being located on the microscope slide 430 during fluid dispensing/aspirating within the microfluidic device 400.

In an alternate embodiment, the vent 480 could be located near the centre of the top surface 425 or anywhere else to allow air to escape from the fluid well insert cavity 424. Positioning the vent 480 near the centre of the top surface 425 of the fluid well insert cavity 424 reduces a likelihood of fluid sloshing up the walls of the fluid well insert cavity 424 and out of the vent 480 during transport of the microfluidic device 400.

In an alternate embodiment, an additional angled vent is provided on the top surface 425 of the closed top fluid well insert 420. This prevents any dust/debris in the environment from falling directly into the fluid well insert cavity 424 because the angled inner wall of the angled vent obstructs access to the fluid well insert cavity 424. Thus, said dust/debris gets collected on the angled inner wall of the angled vents and is prevented from contaminating the biological sample enclosed within the fluid well insert cavity 424. Any ambient light entering the angled vent would similarly be diminished by getting deflected by the angled inner wall of the angled vent.

Figure 10 also shows the base structure 410 which comprises a recess 415 for retaining the microscope slide 430 therein. The base structure 410 also comprises an optical window 440 within the recess 415. The optical window 440 allows imaging of a biological sample located on a top surface of the microscope slide 430 from under the microscope slide 430, when the microscope slide 430 is retained within the recess 415. The optical window 440 could be formed as an open aperture in the base structure 410 or may comprise one or more window materials for transmitting radiation at any desired wavelength, or spectra of wavelengths as described previously.

The base structure 410 also comprises one or more rotatable eccentric cams 455 attached to the base structure 410 and positioned around the recess 415. The one or more rotatable eccentric cams 455 provide a locking/unlocking mechanism for the microscope slide 430 within the microfluidic device 400. During use, the one or more rotatable eccentric cams 455 are rotated to a locked or an unlocked position to respectively engage or disengage with the microscope slide 430 placed within the recess 415 of the base structure 410. When in the locked position, the one or more rotatable eccentric cams 455 push against one or more edges of the microscope slide 430 to firmly hold the microscope slide 430 in place. This prevents any accidental displacement of the microscope slide 430 from the recess 415 when the microfluidic device 400 is in use.

In an alternate embodiment, the base structure 410 further comprises one or more datum lands 416. The one or more datum lands 416 are specially shaped structures which reduce stress concentrations on the microscope slide 430 and thereby prevent chipping of one or more edges of the microscope slide 430 when the microscope slide 430 is locked in place by the one or more eccentric cams 455. During use, when the one or more rotatable eccentric cams 455 are rotated to a locked position, the one or more eccentric cams 455 push against the one or more edges of the microscope slide 430. This in turn pushes the microscope slide 430 against the one or more datum lands 416 on the base structure 410, thus holding the microscope slide 430 firmly in place while distributing the stress evenly over the microscope slide 430.

The microfluidic device 400 is configured such that, when the microscope slide 430 comprising the biological sample is placed in the recess 415 and locked in place by rotating the one or more rotatable eccentric cams 455 to a locked position, an area of the microscope slide 430 comprising the biological sample overlaps with the optical window 440 in the base structure 410, thereby positioning the biological sample to be imaged directly above the optical window 440.

After the microscope slide 430 is locked within the recess 415 in the base structure 410, the closed top fluid well insert 420 is aligned on top of the microscope slide 430 and attached to the base structure 410 using the one or more integrated fittings 490. Preferably, a portion of the microscope slide 430 remains uncovered by, and is visible adjacent to, the closed top fluid well insert 420 when the latter is *in situ,* such that a labelled portion 432 remains visible during the imaging process. As mentioned previously, the labelled portion 432 may then be used to uniquely identify the biological sample contained on the microscope slide 430.

The attachment of the closed top fluid well insert 420 to the base structure 410, fluidically seals the fluid well insert cavity 424 with the top surface of the microscope slide 430. The microfluidic device 400 is further configured such that, when the closed top fluid well insert 420 is attached to the base structure 410, the biological sample on the microscope slide 430 is positioned within the confines of the fluidically sealed fluid well insert cavity 424. Fluids, such as reagents, are then dispensed into and/or aspirated out of the fluidically sealed fluid well insert cavity 424 via the one or more fluid aspirate/dispense ports 470 located on the top surface 425 of the closed top fluid well insert 420 to perform a multiplexed analysis of the biological sample.

Once the multiplexing process is complete, the closed top fluid well insert 420 is detached from the base structure 410 by using the one or more integrated fittings 490. The one or more cams 455 are then rotated to an unlock position so as to disengage with the microscope slide 430 which can then be lifted out of the recess 415 and removed from the microfluidic device 400.

Figure 11 shows a cutaway view of the microfluidic device 400 of Figure 10, where the closed top fluid well insert 420 is attached to the base structure 410. Figure 11 shows a portion of the fluid well insert cavity 424 within the closed top fluid well insert 420 and located under the top surface 425 of the closed top fluid well insert 420.

Figure 11 also shows one fluid aspirate/dispense port 470 and one vent 480 located on the top surface 425 of the closed top fluid well insert 420. In this embodiment, the fluid aspirate/dispense port 470 and the vent 480 are located in an area of the top surface 425 that is not directly above the biological sample. As shown, the fluid aspirate/dispense port 470 and the vent 480 are in fluid communication with the fluid well insert cavity 424.

In this embodiment, the fluid aspirate/dispense port 470 has a funnel shaped configuration such that the wider end of said funnel is integrated with the top surface 425 of the closed top fluid well insert 420 and the narrow end of said funnel is located adjacent to the base of the fluid well insert cavity 424.

One advantage associated with the funnel shaped configuration of the fluid aspirate/dispense port 470 is that it helps in collecting as much residual fluid as possible when the microfluidic device 400 is tilted to aspirate fluid from the fluid well insert cavity 424, thereby resulting in reliable and efficient emptying of the fluid well insert cavity 424.

Another advantage associated with the funnel shaped configuration of the fluid aspirate/dispense port 470 is that, when a pipette tip is inserted into the fluid aspirate/dispense port 470 to aspirate/dispense fluid, the narrow end of the funnel acts as an extension of the pipette tip thereby helping prevent the pipette tip from coming into contact with the biological sample on the microscope slide 430.

When the closed top fluid well insert 420 is placed on top of the locked-in microscope slide 430 and attached to the base structure 410 via the integrated fittings 490, the fluid well insert cavity 424 gets closed at the bottom by the top surface of the microscope slide 430 to form a fluidically sealed fluid well insert cavity 424. The microfluidic device 400 is configured such that, when the closed top fluid well insert 420 is attached to the base structure 410, the biological sample on the top surface of the microscope slide 430 is positioned within the fluidically sealed fluid well insert cavity 424 so that when one or more fluids are dispensed via port 470 into the fluid well insert cavity 424, the one or more fluids come in contact with the biological sample.

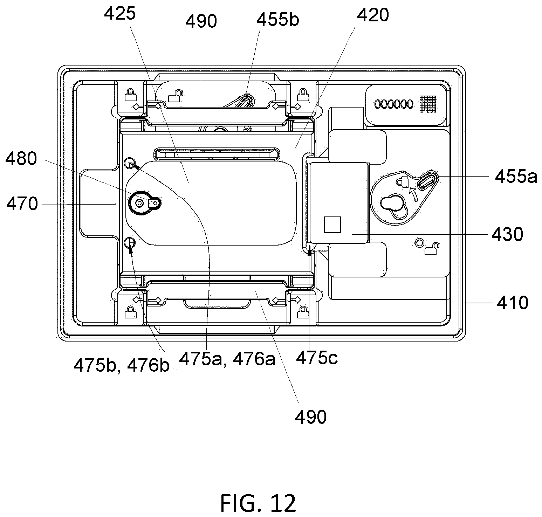

Figure 12 shows a plan view of the microfluidic device 400 of Figure 10 where the closed top fluid well insert 420 is attached to the base structure 410. The base structure 410 comprises one or more optical slide indexing holes 475. The one or more optical slide indexing holes 475 are used to allow optical slide indexing using edge detection techniques, or any other suitable technique. This may be important for multiplexed imaging methods because a series of images are superimposed on each other to create a final image. For example, mechanical indexing using the one or more rotatable eccentric cams 455 can provide a repeatable rough location of the microscope slide 430, while optical slide indexing using the one or more through holes 475 may then provide even higher resolution indexing.

Any of the one or more optical slide indexing holes 475 that gets blocked by the closed top fluid well insert 420 may be unblocked by providing one or more through holes 476 in the closed top fluid well insert 420. Each of said one or more through holes 476 in the closed top fluid well insert 420 is configured to align on top of a respective blocked optical slide indexing hole 475 when the closed top fluid well insert 420 is attached to the base structure 410, thereby forming a continuous channel for light to pass through.

This embodiment shows two rotatable eccentric cams 455a and 455b that are situated adjacent to a first shorter side and a first longer side of the microscope slide 430 respectively. In this embodiment of the invention, the rotatable eccentric cams 455a and 455b have been rotated to a locked position to engage with the microscope slide 430, thereby locking the microscope slide 430 in the base structure 410.

This embodiment also shows three optical slide indexing holes 475a, 475b and 475c that are located on the support structure 410. As shown in Figure 12, the optical slide indexing holes 475a and 475b are located near a second shorter side of the microscope slide 430, and the optical slide indexing hole 475c is located near the labelled portion 432 of the microscope slide 430.

This embodiment also shows two through holes 476a and 476b located on the closed top fluid well insert 420. As shown in Figure 12, the through hole 476a is aligned on top of the optical slide indexing hole 475a. Similarly, the through hole 476b is aligned on top of the optical slide indexing hole 475b. No through hole is required for the optical slide indexing hole 475c as it is not obstructed by the closed top fluid well insert 420.

Other embodiments of the invention that would be readily apparent to the skilled person include microfluidic devices comprising one or more rotatable eccentric cams 455, one or more integrated fittings 490 integrated to the closed top fluid well insert 420, one or more fluid aspirate/dispense ports 470, one or more vents 480, one or more optical slide indexing holes 475 and/or one or more through holes 476. Rotatable eccentric cams 455 may be provided with edge features (e.g. with resilient materials and/or shaped features on the periphery thereof) for biasing against an edge of the microscope slide 430.

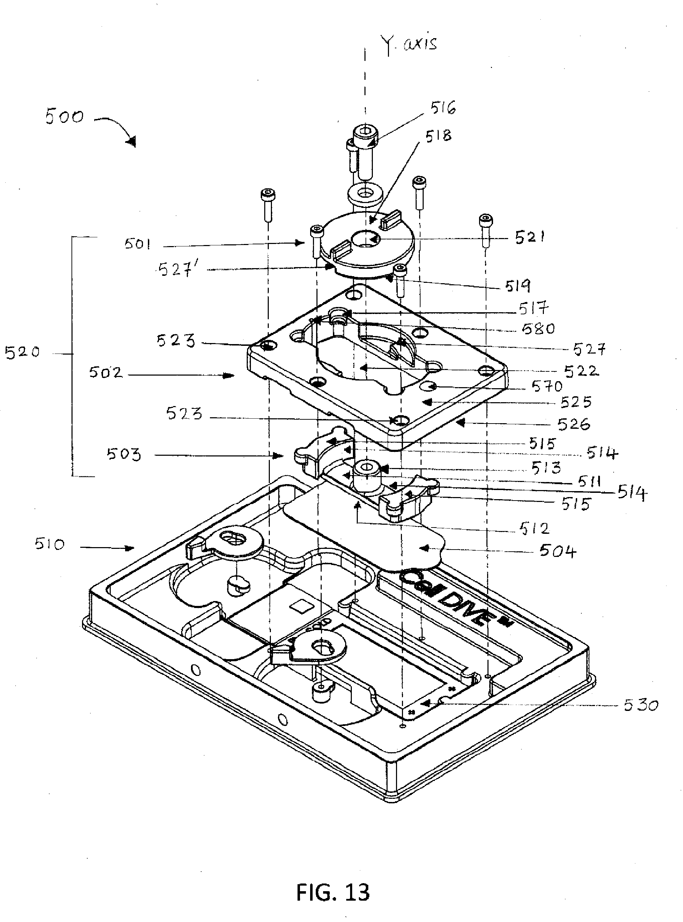

Figure 13 shows a microfluidic device 500 in an exploded view in accordance with a third embodiment of the present invention, showing a fluid well insert 520 configured to provide a fluid well cavity 524 with an adjustable and/or switchable volume.

The fluid well insert 520 comprises a rotatable dial 501, a fluid well insert frame 502, and a diaphragm plate 503. The fluid well insert frame 502 comprises a top surface 525, a bottom surface 526 and a through cavity 522 within the fluid well insert frame 502. The through cavity 522 of the fluid well insert frame 502 extends through the top surface 525 and the bottom surface 526 of the fluid well insert frame 502 along a common vertical axis Y of the fluid well insert 520. The fluid well insert frame 502 is configured such that it can retain the rotatable dial 501 in the through cavity 522 adjacent to the top surface 525. The fluid well insert frame 502 is also configured such that it can retain the diaphragm plate 503 in the through cavity 522 adjacent to the bottom surface 526.

The fluid well insert 520 also comprises one or more fluid aspirate/dispense ports 570 and one or more vents 580 located on the top surface 525 of the fluid well insert frame 502. The one or more aspirate dispense ports 570 and the one or more vents 580 are similar to those described in one or more previous embodiments. In this embodiment, the fluid well insert 520 has one fluid aspirate/dispense port 570 and one vent 580 located diametrically opposite to each other and adjacent to the through cavity 522 of the fluid well insert frame 502.

The through cavity 522 of the fluid well insert frame 502 comprises one or more ramps/cams 527 on an inner wall of the through cavity 522 and adjacent to the top surface 525 of the fluid well insert frame 502.

The rotatable dial 501 comprises a top surface 518, a bottom surface 519 and a through hole 521 within the rotatable dial 501. The through hole 521 extends from the top surface 518 to the bottom surface 519 of the rotatable dial 501 along the common vertical axis Y of the fluid well insert 520. The rotatable dial 501 is configured such that it can be retained within the through cavity 522 of the fluid well insert frame 502.

The rotatable dial 501 also comprises one or more ramps/cams 527' located on the bottom surface 519 near the periphery of the rotatable dial 501. The one or more ramps/cams 527' are complimentary to the one or more ramps/cams 527 in the through cavity 522 of the fluid well insert frame 502. When the rotatable dial 501 is placed into the through cavity 522 of the fluid well insert frame 502, the one or more ramps/cams 527' and the one or more ramps/cams 527 are slidably engaged with each other, allowing the rotatable dial 501 to rotate within the through cavity 522. The rotatable dial 501 is rotatable around the common vertical axis Y of the fluid well insert 520 in a horizontal plane that is perpendicular to the common vertical axis Y.

The diaphragm plate 503 comprises a top surface 511, a bottom surface 512 and a central connector 513 located on the top surface 511 of the diaphragm plate 503. The diaphragm plate 503 is configured to be retained within the through cavity 522 of the fluid well insert frame 502. The diaphragm plate 503 also comprises a plurality of protrusions 515 located on the top surface 511 on the outer end portions of the diaphragm plate 503, thereby forming a plurality of arcuate shaped cavity portions 514 defined between the central connector 513 and the respective outer end portions of the diaphragm plate 503.

In one assembled configuration of the fluid well insert 520, the diaphragm plate 503 and the rotatable dial 501 are retained within the through cavity 522 of the fluid well insert frame 502. The rotatable dial 501 is positioned on top of the diaphragm plate 503 such that the bottom surface 519 of the rotatable dial 501 faces the top surface 511 of the diaphragm plate 503. The rotatable dial 501, the diaphragm plate 503 and the fluid well insert frame 502 are aligned along the common vertical axis Y and are connected to each other to assemble the fluid well insert 520. In this embodiment, a rivet 516 is used to connect the rotatable dial 501 to the diaphragm plate 503 through the fluid well insert frame 502 to assemble the fluid well insert 520. The stem of the rivet 516 is lodged into a central bore of the central connector 513 to affix the rotatable dial 501 to the diaphragm plate 503. In an alternate embodiment, any other suitable technique could be employed to connect various components of the fluid well insert 520 to each other.

In this assembled configuration of the fluid well insert 520, the plurality of protrusions 515 on the diaphragm plate 503 are slidably engaged with the inner wall of the through cavity 522. The plurality of arcuate shaped cavity portions 514 on the diaphragm plate 503 are aligned with the one or more ramps/cams 527 provided in the through cavity 522 of the fluid well insert frame 503 along a common horizontal axis, thereby forming an annular recess (not shown) within the through cavity 522 of the fluid well insert frame 502.

The rotatable dial 501 is retained within said annular recess and connected to the central connector 513 of the diaphragm plate 503. The rotatable dial 501 is also engaged with the one or more ramps/cams 527 provided within the through cavity 522 of the fluid well insert frame 502 through the one or more ramps/cams 527' provided on the bottom surface 519 of the rotatable dial 501.

The fluid well insert 520 is closed by fluidically sealing an opening of the through cavity 522 on the bottom surface 526 of the fluid well insert 520 with a fluid well insert lid 504. Further, at least a portion of the bottom surface 512 of the diaphragm plate 503 is affixed to the fluid well insert lid 504, thereby connecting the fluid well insert lid 504 to the rotatable dial 501 via the central connector 513 of the diaphragm plate 503. The fluid well insert lid 504 could be affixed to the bottom surface 512 by using glue or a mechanical fastener or in any other suitable manner.

The base structure 510 comprises features that are similar to the features of the base structures described in one or more previous embodiments, such as, a recess to retain a microscope slide 530 therein, one or more eccentric cams to hold the microscope slide 530 in place, an optical window to image the biological sample on the microscope slide 530 and one or more optical slide indexing holes. In this embodiment, the fluid well insert 520 is affixed to the base structure 510 by using six screws 523 along the periphery of said fluid well insert 520. In an alternate embodiment, the fluid well insert 520 could comprise one or more integrated snap fittings and thus could be attached to the base structure 510 using said integrated snap fitting as described in one or more previous embodiments.

In use, the microscope slide 530 with a biological sample thereon is locked in place within the base structure 510 in a similar fashion as described in one or more previous embodiments. Thereafter, the fluid well insert 520 that has been closed using the fluid well insert lid 504 is affixed to the base structure 510, thereby defining a fluid well cavity 524 enclosed between the microscope slide 530 and the fluid well insert lid 504.

The microfluidic device 500 is configured such that said fluid well cavity 524 is fluidically sealed. The microfluidic device 500 is also configured such that the biological sample contained on the microscope slide 530 is enclosed within said fluid well cavity 524 while remaining within the optical window on the base structure 510 so that the biological sample could be imaged.

In this embodiment, the fluid well insert 520 is configured such that said fluid well cavity 524 has an adjustable and/or switchable volume and the fluid well insert lid 504 comprises a flexible diaphragm 504. The fluid well insert 520 is closed by fluidically sealing the opening of the through cavity 522 on the bottom surface 526 of the fluid well insert 520 by said diaphragm 504. The diaphragm 504 is preferably made of a flexible and/or a stretchable material such that when a pressure is applied on a surface of the diaphragm 504, the diaphragm 504 is reversibly deformed to form a hollow cavity/protrusion, thereby providing a mechanism for adjusting and/or switching the volume of said fluid well cavity 524. One suitable material to make such diaphragm is rubber.

In this embodiment, the volume of said fluid well cavity 524 is adjusted by rotating the rotatable dial 501 on the fluid well insert 520. The microfluidic device 500 is configured such that when the rotatable dial 501 is turned, the diaphragm plate 503 connected to the rotatable dial 501 moves within the through cavity 522 in an upward or a downward direction along the common vertical axis Y. Due to the diaphragm 504 being affixed to the bottom surface 512 of the diaphragm plate 503, an upward movement of the diaphragm plate 503 causes the diaphragm 504 to be pulled upwards, thereby increasing the volume of said fluid well cavity 524. Similarly, a downward movement of the diaphragm plate 503 causes the diaphragm to be pushed downwards, thereby decreasing the volume of said fluid well cavity 524.

The fluid well insert 520 is configured such that when the microfluidic device 500 is in use, the fluid well insert lid 504 is prevented from coming into contact with the biological sample contained on the microscope slide 530, to prevent contamination and/or damage to the biological sample. Thus, the fluid well insert 520 is configured such that the lowest volume achievable by turning the rotatable dial 501 maintains the fluid well insert lid 504 at a sufficiently safe distance from the biological sample. This embodiment utilises a plurality of protuberances 517 provided on the inner wall of the through cavity 522 that provide a barrier to stop the downward movement of the diaphragm plate 503 beyond said plurality of protuberances 517. In an alternate embodiment, the restriction on the range of the downward movement of the diaphragm plate 503 could be achieved by using various different techniques that would be readily apparent to the skilled person.

Also, as would be apparent to the skilled person, the microfluidic device 500 could be configured such that the volume of the fluid well cavity 524 is adjustable over a range of volume selections or to a plurality of discrete predefined/pre-set volume selections.

In an alternate embodiment, the fluid well insert lid 504 could include one or more structural features that allow the volume of the fluid well cavity 524 to increase or decrease. One such structural feature could be for example, one or more folding bellows provided in the fluid well insert lid 504, such that opening and closing of said one or more folding bellows would provide a mechanism to adjust the volume of the fluid well cavity 524.

One advantage of having an adjustable and/or switchable volume feature as described above in regard to the microfluidic device 500 is that, it allows effective utilisation of smaller volumes of fluids to cover a biological sample contained on the microscope slide evenly. This is achieved by reducing the volume of the fluid well cavity 524 such that air within the fluid well cavity 524 is diminished and nearly all the space within the fluid well cavity 524 is occupied by said fluid. This is highly beneficial when working with expensive fluids like an antibody solution.

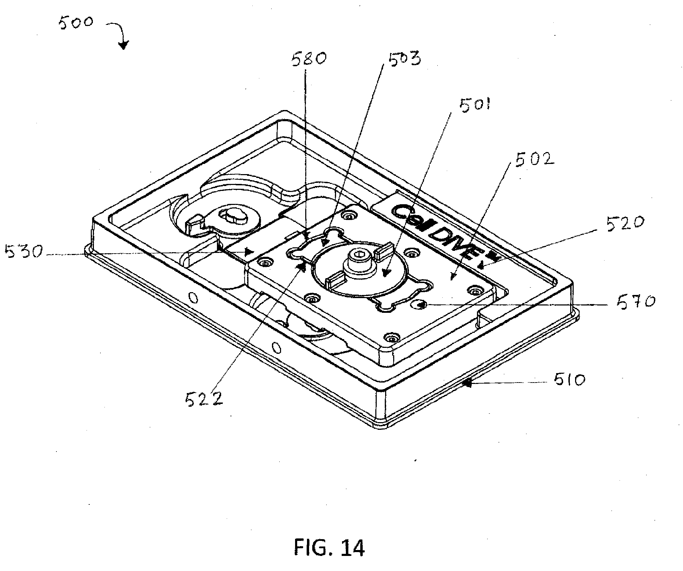

Figure 14 shows a plan view of the microfluidic device 500 of Figure 13 showing the fluid well insert 520 attached to the base structure 510 and holding the microscope slide 530 there between. The rotatable dial 501 and the diaphragm plate 503 are retained within the through cavity 522 of the fluid well insert frame 502 to form the fluid well insert 520.

In various embodiments, a device of the present invention can accommodate fluid volumes ranging from 1 µl to 7000µl within a fluid well cavity of the device while protecting the biological sample and any fluids therein from environmental contaminants that may otherwise be introduced were the fluid well to be open.

In various embodiments, a device of the present invention can accommodate fluid through the one or more fluid aspirate/dispense ports by utilizing a non-contact dispenser system. In embodiments, a distal end of a non-contact dispenser, such as a pipette tip, dispenses fluids, such as reagents, into the fluid aspirate/dispense port without physically touching the fluid aspirate/dispense port. In this embodiment, the fluid aspirate/dispense port 470 has an angled or a funnel shaped configuration such that the wider end of said funnel is integrated with the top surface 425 of the closed top fluid well insert 420 and the narrow end of said funnel is located adjacent to the base of the fluid well insert cavity 424. For example, such ports 170, 180 (or 470) can be conically-shaped to accommodate a pipette or syringe therein, as are known in the art, to introduce or extract fluid(s) into or from the microfluidic device, for example, as part of a robotic computer controlled non-contact dispenser system. Such embodiments can reduce cross-contamination and excessive discarding of pipette tips.

In embodiments, a non-contact dispenser system can prevent pressure build-up inside the fluid well insert cavity 424 potentially causing contamination or leakage from the microfluidic device which is ameliorated by removing air from the one or more vents 480 commensurate with or greater than an amount of fluid introduced into the fluid well insert cavity 424 by the corresponding fluid aspirate/dispense ports 470. In an embodiment, the non-contact dispenser system is a pipette or syringe that removes air by creating negative pressure at the one or more vents 480 commensurate with or greater than an amount of fluid introduced into the fluid well insert cavity 424 via the corresponding fluid aspirate/dispense ports 470. Removal of the air via a vent 480 can occur before, during or after introduction of the fluid via a corresponding fluid aspirate/dispense port 470.

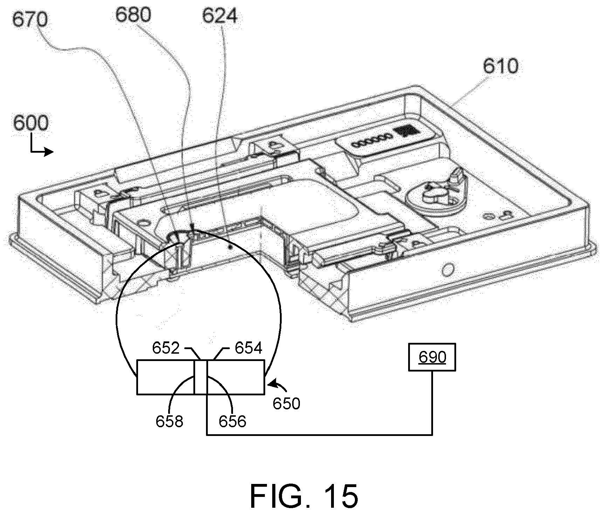

Figure 15 shows an embodiment where the invention provides microfluidic device 600 further comprising a non-contact dispenser system 610 comprising a reciprocating syringe 650 that withdraws an amount of air from a fluid well insert cavity 624 via contact sealing with a vent 680 by vacuum/negative pressure that is commensurate with or greater than an amount of fluid introduced into the fluid well insert cavity 624 via a corresponding fluid aspirate/dispense port 670. In embodiments, a non-contact dispenser system provides a reciprocating syringe 650 having a common movable plunger 652 within a single barrel 654, with a first plunger side 656 receiving air from the fluid well insert cavity 624 via vent 680 and an opposing second plunger side 658 delivering fluid to the fluid well insert cavity 624 via a corresponding fluid aspirate/dispense port 670. Movement of such a reciprocating plunger 652 can be achieved either manually or automated with a computer processing unit (CPU) implemented volume monitoring and controller system 690. This aspect of the invention can be used to reduce contamination as well as the formation of bubbles, which can otherwise cause non-uniform staining of the biological sample and thereby degrade images by introducing random image artefacts. In embodiments, a non-contact dispenser system and associated tubing is provided in a kit with the microfluidic device of the present invention.

In alternative embodiments, the non-contact dispensing system can include a dispense syringe and vacuum syringe as separately actuated mechanisms, either manually or electronically controlled. This embodiment allows independent control of each syringe, such that removing air from the one or more vents 480 by negative pressure can draw a greater volume of air than fluid introduced into the fluid well insert cavity 424 by the corresponding fluid aspirate/dispense ports 470, and also allows vacuum to continue past the point when dispensing has ended, thus ensuring that all the fluid is dispensed into the chamber.

In alternative embodiments, the non-contact dispensing system can include a "teeter-totter" mechanism that has a syringe or pipette on each of the ends. The leg length of each opposing teeter-totter end can be adjusted to change the ratio of dispensed fluid to vacuumed air. For example, the teeter-totter can have one short leg end (liquid dispense end) and one long leg end (air removal end). In embodiments where the legs, e.g. syringe barrel bores, have the same diameter, the longer end will provide a greater volume displacement than the shorter end, which ensures that all the fluid gets dispensed into the chamber.

Various aspects and embodiments of the present invention have thus been described herein. Nevertheless, many variations thereof will be apparent to the skilled person, and it is intended that these fall within the scope of the invention.

For example, a heater and/or cooler may be provided within the fluid well insert or elsewhere in the microfluidic device to enable temperature control therein to be provided. For example, one or more heaters and/or thermo-electric coolers may be incorporated.

Various embodiments of fluid well inserts may also be designed, e.g. for holding one or more volumes of reagents. For example, by including reagent wells, an insert may be provided that is pre-loaded with some of the particular stains needed for the automated multiplexing process that will be carried out on it. Well-plate structures located at edges of an insert could thus be provided which are pre-filled and covered, for example, with a pierceable film.

Moreover, whilst embodiments of the invention refer to use with microscope slides, those skilled in the art would also be aware that, for example, a tissue microarray may also be used for imaging purposes within embodiments of the invention. Furthermore, in various embodiments, a fluid well insert may be provided having a plurality of separate fluid well insert cavities provided therein, each optionally provided with a respective fluid guide structure (e.g. in a well-plate, or well-plate like, format).

However, the scope of the invention is only limited by the appended claims, when correctly interpreted with regard to the full disclosure of the present application.

## Claims

1. A microfluidic device (100, 400, 500, 600) for image multiplexing, the microfluidic device comprising:
a base structure (110, 410) comprising an optical window (140, 440);
a fluid well insert (120, 420) being configured to be coupled to the base structure (110, 410) and to retain a microscope slide (130) for mounting of a biological sample (150) within the microfluidic device (100), said fluid well insert (120, 420) being further configured to provide a fluid to said biological sample (150); and
a fluid well insert lid (160) coupled to the fluid well insert (120) or the fluid well insert (120) comprising a fluid well insert lid (160).

2. The microfluidic device (100, 400, 500, 600) of claim 1, wherein the fluid well insert lid (160) comprises an opaque cover material and/or a fluid input port (170) and a fluid aspiration port (180) and/or a fluid aspirate/dispense port (610, 470).

3. The microfluidic device (100, 400, 500, 600) of any preceding claim, wherein the base structure (110) further comprises at least one quick release coupling mechanism (190) for releasably attaching the fluid well insert (120) to the base structure (110).

4. The microfluidic device (100, 400, 500, 600) of claim 3, comprising a plurality of quick release coupling mechanisms (190) each comprising a respective rotatable lug (192), and wherein the rotatable lugs (192) are configured to engage with a respective ledge portion (122) provided on said fluid well insert (120), wherein the rotatable lugs (192) are particularly provided on respective spring clamps (194, 196) attached to the base structure (110).

5. The microfluidic device (100, 400, 500, 600) of any preceding claim, wherein the optical window (140) is substantially transparent to electromagnetic radiation in at least one of the infrared, near-infrared, visible and/or ultraviolet spectra.

6. The microfluidic device (100, 400, 500, 600) of any preceding claim, wherein the fluid well insert (120) further comprises a fluid well insert cavity (124) for providing a fluid in contact with the biological sample (150), wherein the fluid well insert (120) particularly further comprises one or more of: a fluid dispenser cavity (172) in fluid communication with the fluid well insert cavity (124) and/or a fluid aspiration cavity (182) in fluid communication with the fluid well insert cavity (124), the fluid dispenser cavity (172) particularly comprising at least one off axis fluid entry point therein, wherein the fluid well insert cavity (124) and/or the fluid well insert (120) particularly further comprises at least one fluid guide structure (184) configured to channel fluid into the fluid aspiration cavity (182) as the microfluidic device is tilted, wherein the fluid aspiration cavity (182) and/or the at least one fluid guide structure (184) particularly comprise a shaped floor portion configured to enable the fluid aspiration cavity (182) to retain fluid therein should the microfluidic device be untilted, wherein the fluid aspiration cavity (182) and/or the at least one fluid guide structure (184) particularly comprise a fluid dam structure (186) therein, the fluid dam structure (186) particularly having a substantially triangular cross sectional shape particularly providing a sloped surface portion thereof angled at an angle (α) from about 10° to about 15° with respect to a floor portion of the fluid well insert (120).

7. The microfluidic device (100, 400, 500, 600) of any preceding claim, wherein the fluid well insert (120) is configured to enable a labelled portion (132) of the microscope slide (130) to remain visible when in use, and/or wherein the base structure (110) further comprises one or more fiducial features (112) provided thereon.

8. The microfluidic device (100, 400, 500, 600) of any preceding claim, further comprising one or more heater and/or cooler operable to control the temperature thereof.

9. The microfluidic device (100, 400, 500, 600) of any preceding claim, further comprising one or more reagent wells, optionally provided within the fluid well insert (120), wherein said one or more reagent wells are pre-loaded with stains needed for an automated multiplexing process and particularly covered, optionally with a pierceable film.

10. The microfluidic device (100, 400, 500) of any preceding claim, wherein the fluid well insert lid (160) and the fluid well insert (120) are formed as a single component in the form of a closed top fluid well insert (420), wherein the closed top fluid well insert (420) particularly further comprises at least one integral snap fitting (490) to releasably couple the closed top fluid well insert (420) to the base structure (410) and/or a fluid aspirate/dispense port (470) and a vent (480) located on a top surface (425) of the closed top fluid well insert (420).

11. The microfluidic device (100, 400, 500, 600) of claim 10, comprising at least one fluid aspirate/dispense port (470) and/or at least one vent (480) that is offset from a central position where the biological sample (150) is to be located, and/or comprising at least one rotatable eccentric cam (455) attached to the base structure (410), wherein at least one rotatable eccentric cam (455) is/are configured to engage with the microscope slide (430) so as to releasably lock the microscope slide (430) in place on the base structure (410).

12. The microfluidic device (100, 400, 500, 600) of any preceding claim, wherein the microscope slide (530) and the fluid well insert lid (504) define a fluid well cavity (524), said fluid well cavity (524) having an adjustable and/or switchable volume.

13. The microfluidic device (100, 400, 500, 600) of claim 12, comprising a fluid well insert (520) that is configured to provide the fluid well cavity (524) with the adjustable and/or switchable volume, wherein the fluid well insert particularly (520) comprises:
a rotatable dial (501) actuatable to select a volume of the fluid well cavity (524);
a fluid well insert frame (502) comprising a through cavity (522) and particularly further comprising or more fluid aspirate/dispense ports (570) and/or one or more vents (580); and
a diaphragm plate (503),
wherein the fluid well insert frame (502) is configured to retain the rotatable dial (501) and the diaphragm plate (503) substantially within the though cavity (522).

14. The microfluidic device (100, 400, 500, 600) of any one of claims 12 and 13, wherein the volume of the fluid well cavity (524) is adjustable and/or switchable within a volume range of 1 µl - 7000µl.

15. The microfluidic device (100, 400, 500, 600) of any preceding claim, further comprising a reciprocating syringe (650) having a common movable plunger (652) within a single barrel (654), with a first plunger side (656) receiving air from the fluid well insert cavity (624) via a vent (680) and an opposing second plunger side (658) delivering fluid to the fluid well insert cavity (624) via a corresponding fluid aspirate/dispense port (670), wherein the movable plunger (652) is particularly controlled by a computer processing unit (CPU) implemented volume monitoring and controller system (690).
